# EUROPEAN PATENT SPECIFICATION

(11) **EP 3 766 521 B1**
(45) Date of publication and mention of the grant of the patent: **25.06.2025**
(21) Application number: 19767854.3
(22) Date of filing: 22.02.2019
(51) Int. Cl.: A61K 51/00, A61K 31/198, A61K 31/28, A61P 35/00, C07B 59/00, A61K 51/04

(54) **PHARMACEUTICAL COMPOSITION CONTAINING 211AT-LABELED AMINO ACID DERIVATIVE, AND METHOD FOR PRODUCING SAID PHARMACEUTICAL COMPOSITION**
PHARMAZEUTISCHE ZUSAMMENSETZUNG MIT 211AT-MARKIERTEM AMINOSÄUREDERIVAT UND VERFAHREN ZUR HERSTELLUNG DIESER PHARMAZEUTISCHEN ZUSAMMENSETZUNG
COMPOSITION PHARMACEUTIQUE CONTENANT UN DÉRIVÉ D'ACIDE AMINÉ MARQUÉ AU 211AT, ET PROCÉDÉ POUR PRODUIRE LADITE COMPOSITION PHARMACEUTIQUE

(30) Priority: 15.03.2018 JP 2018048562
(43) Date of publication of application: 20.01.2021
(73) Proprietor: Osaka University, Suita-shi, Osaka 565-0871 (JP)
(72) Inventor: FUKASE, Koichi, Suita-shi, Osaka 565-0871 (JP); SHINOHARA, Atsushi, Suita-shi, Osaka 565-0871 (JP); KANAI, Yoshikatsu, Suita-shi, Osaka 565-0871 (JP); KABAYAMA, Kazuya, Suita-shi, Osaka 565-0871 (JP); KANEDA, Kazuko, Suita-shi, Osaka 565-0871 (JP); ZHANG, Zijian, Suita-shi, Osaka 565-0871 (JP); HATAZAWA, Jun, Suita-shi, Osaka 565-0871 (JP); SHIRAKAMI, Yoshifumi, Suita-shi, Osaka 565-0871 (JP); SHIMOYAMA, Atsushi, Suita-shi, Osaka 565-0871 (JP); MANABE, Yoshiyuki, Suita-shi, Osaka 565-0871 (JP)
(74) Representative: Symbiosis IP Limited
(86) International application number: PCT/JP2019/006800
(87) International publication number: WO 2019/176505

(56) References cited:
- EP-A1- 3 663 307
- WO-A2-2007/079953
- WO-A2-2011/147762
- DE-A1- 10 127 835
- US-A1- 2007 128 108
- HARRISON A.: "The Application of 211 At in Experimental Tumour Therapy", RADIOCHIMICA ACTA, vol. 47, no. 2-3, 1 January 1989 (1989-01-01), DE, pages 157 - 161, XP055773159, ISSN: 0033-8230, Retrieved from the Internet <URL:http://dx.doi.org/10.1524/ract.1989.47.23.157> DOI: 10.1524/ract.1989.47.23.157
- OHSHIMA YASUHIRO: "Development of 211At-labeled alpha-emitting radiopharmaceutical", INTERNET CITATION, 16 January 2018 (2018-01-16), XP009522907, Retrieved from the Internet <URL:https://kaken.nii.ac.jp/ja/report/KAKENHI-PROJECT-16K21603/16K216032016hokoku>
- AYED TAHRA ET AL: "211At-labeled agents for alpha-immunotherapy: On thein vivostability of astatine-agent bonds", EUROPEAN JOURNAL OF MEDICINAL CHEMISTRY, ELSEVIER, AMSTERDAM, NL, vol. 116, 29 March 2016 (2016-03-29), pages 156 - 164, XP029536403, ISSN: 0223-5234, DOI: 10.1016/J.EJMECH.2016.03.082
- VISSER G W M ET AL: "The preparation and stability of astatotyrosine and astato-iodotyrosine", INTERNATIONAL JOURNAL OF APPLIED RADIATION AND ISOTOPS, PERGAMON PRESS, NEW YORK, NY, US, vol. 30, no. 12, 1 December 1979 (1979-12-01), pages 749 - 752, XP024717391, ISSN: 0020-708X, [retrieved on 19791201], DOI: 10.1016/0020-708X(79)90154-6
- CASTILLO MELEÁN JOHNNY ET AL: "Stereoselective radiosynthesis ofl- andd-3-[18F]fluoro-[alpha]-methyltyrosine", JOURNAL OF FLUORINE CHEMISTRY, ELSEVIER, NL, vol. 178, 18 July 2015 (2015-07-18), pages 202 - 207, XP029283774, ISSN: 0022-1139, DOI: 10.1016/J.JFLUCHEM.2015.07.015
- BLOOMER WILLIAM ET AL: "Experimental Therapeutics Using Alpha Particles", RADIOCHIMICA ACTA, OLDENBOURG WISSENSCHAFTSVERLAG GMBH MUNICH, DE, vol. 47, no. 2-3, 30 November 1988 (1988-11-30), pages 149 - 151, XP009522906, ISSN: 0033-8230, DOI: 10.1524/RACT.1989.47.23.149
- DATABASE CAPLUS [online] ACS; 1 June 1989 (1989-06-01), BLOOMER WILLIAM D. ET AL: "Experimental Therapeutics Using Alpha Particles - CAS RN 125010-26-6", XP055935065, Database accession no. 1990:73005
- OVERWIJK WILLEM W. ET AL: "B16 as a Mouse Model for Human Melanoma", NANOSCIENCE : VOLUME 5 / EDITORS: P. JOHN THOMAS (BANGOR UNIVERSITY, UK), NEERISH REVAPRASADU (UNIVERSITY OF ZULULAND, SOUTH AFRICA), vol. 39, no. 1, 1 October 2000 (2000-10-01), US, XP055834067, ISSN: 1934-3671, ISBN: 978-1-78801-387-1, DOI: 10.1002/0471142735.im2001s39
- CHIEN HUAN-CHIEH ET AL: "Reevaluating the Substrate Specificity of the L-Type Amino Acid Transporter (LAT1)", JOURNAL OF MEDICINAL CHEMISTRY, vol. 61, no. 16, 23 August 2018 (2018-08-23), US, pages 7358 - 7373, XP055893348, ISSN: 0022-2623, DOI: 10.1021/acs.jmedchem.8b01007
- BLOOMER WILLIAM, MCLAUGHLIN WILLIAM: "Experimental Therapeutics Using Alpha Particles", RADIOCHIMICA ACTA, vol. 47, no. 2-3, 1989, pages 149 - 151, XP009522906, DOI: 10.1524/ract.1989.47.23.149
- VISSER, GERARD W. M. ET AL.: "The Preparation and Stability of Astatotyrosine and Astato-iodotyrosine", THE INTERNATIONAL JOURNAL OF APPLIED RADIATION AND ISOTOPES, vol. 30, no. 12, December 1979 (1979-12-01), pages 749 - 752, XP024717391
- OHSHIMA YASUHIRO: "Development of 211At-labeled alpha-emitting radiopharmaceutical", 16 January 2018 (2018-01-16), XP009522907, Retrieved from the Internet <URL:https://kaken.nii.ac.jp/ja/report/KAKENHI-PROJECT-16K21603/16K216032016hokoku>
- SHIKANO, NAOTO ET AL.: "Isoform selectivity of 3-125I-iodo-alpha-methyl-L-tyrosine membrane transport in human L-type amino acid transporters", THE JOURNAL OF NUCLEAR MEDICINE, vol. 44, no. 2, February 2003 (2003-02-01), pages 244 - 246, XP002343137
- KANAI YOSHIKATSU: "Cancer Drug Delivery targeting amino acid Transporters", DRUG DELIVERY SYSTEM, vol. 27, no. 5, 2012, pages 342 - 349, XP055730588
- ISHIOKA NORIKO: "The Therapeutic effect of alpha-ray on malignant pheochromocytoma, Basic evaluation of 211At-MABG as a target isotope therapeutic agent", ISOTOPE NEWS, 2017, pages 14 - 18, XP009522908, ISSN: 0285-5518

## Description

### Technical Field

The present invention relates to a pharmaceutical composition for use in the treatment of cancer, the composition including a ²¹¹At-labeled amino acid derivative, and a method for producing the derivative.

### Background Art

Among amino acid transporters which are membrane proteins required for cellular uptake of neutral branched chain amino acids and aromatic amino acids including a number of essential amino acids, a large neutral amino acid transporter referred to as L-type amino acid transporter 1 (LAT1) is known to function as an uptake port of amino acids in cancer cells. LAT1, which is absent in normal cells in most tissues, is specifically expressed in cancer cells and has a role in supplying amino acids as nutrients in cancer tissues (Non Patent Literature 1).

Till now, a LAT1 inhibitor has been developed with an aim of regression of cancer tissues by functionally inhibiting LAT1 specifically expressed in cancer cells and thereby inducing nutrient starvation in a cancer-specific manner. However, although the LAT1 inhibitor can be applicable and widely effective in various kinds of cancer, it has a short in-vivo half-life and acts by inducing nutrient starvation in cancer cells, thus there is a disadvantage that the LAT1 inhibitor needs to be continuously administered for a certain period of time.

Further, α-methyltyrosine (AMT) is known as an amino acid which is taken up more by the cancer cell-specific LAT1 than the amino acid transporters in normal cells. Conventionally, α-methyltyrosine having an affinity with LAT1 is labeled with ¹⁸F and the resulting fluorine-α-methyltyrosine is used as an imaging probe for positron emission tomography (PET) (Non Patent Literature 2).

On the other hand, for advanced cancer or intractable cancer that is difficult to be treated by a surgical operation, the nuclear medicine therapy has been attempted in which agents including radioactive elements are administered to a patient and the agents accumulated in an affected site emit radioactive rays in the body to kill the cancer cells. Conventionally, as such an agent, a compound having iodine (¹³¹I) has been used. However, β-rays emitted from iodine have high penetrating properties despite having low energy, thus there is a risk that β-rays with insufficient cell-killing activity may still cause a damage to the surrounding normal cells.

In contrast, when nuclides emitting α-rays having lower penetrating properties and higher energy than β-rays are used, the target cancer cells can be expected to be effectively treated by the high cell-killing activity, while minimizing the effects on the normal cells. However, a therapeutic agent using such an α-ray emitting nuclide is left with problems such as a production technique of the α-ray nuclide itself and stability of the compound, making practical application of such a therapeutic agent premature as it is.

Patent Literature 1 discloses radioactively labelled 3-O-methyl-6-halogen-L-DOPA, which is not an α-alkyl tyrosine derivative of Formula (I) below, for treating cancer.

Non Patent Literature 3 discloses ²¹¹At-antibody conjugates for treating cancer, but this document is silent with regard to ²¹¹At-labeling of tyrosine or tyrosine derivatives.

Each of Non Patent Literature 4, Non Patent Literature 5, Patent Literature 2 and Patent Literature 3 discloses ²¹¹At-labeled phenylalanine derivatives for treating cancer.

Non Patent Literature 6 discloses ²¹¹At-labelled tyrosine, but this document fails to disclose any α-alkyl tyrosine compounds.

Non Patent Literature 7 discloses a ¹⁸F-labelled α-alkyl tyrosine derivative, but does not disclose any ²¹¹At-labelled compounds.

Non Patent Literature 8 discloses ²¹¹At-alpha-methyltyrosine (²¹¹At-AMT) for use in the treatment of B16 melanoma cells.

Non Patent Literature 9 discloses treatment protocols using B16 melanoma cells in *in vivo* mouse models for human melanoma.

Patent Literature 4 discloses radiopharmaceutical compositions comprising a radiolabeled protein and one or more stabilizer such as ascorbic acid.

### Citation List

### Non Patent Literature

Non Patent Literature 1: Kanai et al., J. Biol. Chem. 273: 23629, 1998
Non Patent Literature 2: Kaira et al., Clin Cancer Res. 13: 6369-6378, 2007
Non Patent Literature 3: Harrison A., Radiochimica Acta 47, 157-161.
Non Patent Literature 4: Yasuhiro., Internet Citation (https://kaken.nii.ac.jp/ja/report/KAKENHI-PROJECT-16K21603/16K216032016hokoku).
Non Patent Literature 5: Ayed et al., European Journal of Medicinal Chemistry 116 (2016) 156-164.
Non Patent Literature 6: Visser et al., International Journal of Applied Radiation and Isotopes 30, 749-752.
Non Patent Literature 7: Meleán et al., Journal of Fluorine Chemistry 178 (2015) 202-207.
Non Patent Literature 8: Bloomer et al., Radiochimica Acta 47, 149-151 (1989).
Non Patent Literature 9: Overwijk et al., "B16 as a Mouse Model for Human Melanoma". Nanoscience: Volume 5 / editors: P. John Thomas (Bangor University, UK), Revaprasadu (University of Zululand, SA), vol. 39, no. 1, 1 October 2000.

### Patent Literature

Patent Literature 1: DE 101 27 835 A1.
Patent Literature 2: US 2007/128108 A1.
Patent Literature 3: WO 2007/079953 A2.
Patent Literature 4: WO 2011/147762 A2.

### Summary of Invention

### Technical Problem

Under such a circumstance, an object of the present invention is to develop a compound which is highly accumulated in cancer cells and capable of emitting an α-ray and provide a pharmaceutical composition for cancer treatment including the compound. Further, another object is to provide a method for producing such a compound.

### Solution to Problem

As a result of intensive studies intended to solve the above problems, the present inventors have found a method for synthesizing a new compound in which an amino acid derivative having an affinity with an amino acid transporter LAT1 is labeled with astatine-211 (²¹¹At), which is a radioactive nuclide that emits an α-ray and has a relatively short half-life. The present inventors have found that the compound is highly accumulated in cancer cells and exhibits an extremely excellent tumor growth inhibitory effect with fewer side effects such as body weight loss. Further, the present inventors have also found that, when the compound is used in combination with ascorbic acid, which is a reducing agent, the compound can be stably present in the living body for a long period of time, making it possible to improve accumulation of the compound in cancer cells. The present invention has been completed on the basis of these findings.

The present invention, in its various aspects, is as set out in the accompanying claims.

That is, according to an aspect of the present invention, provided are:
<1> A pharmaceutical composition for use in the treatment of cancer, comprising one or more compounds having a structure represented by a following formula (I)or a pharmaceutically acceptable salt thereof, 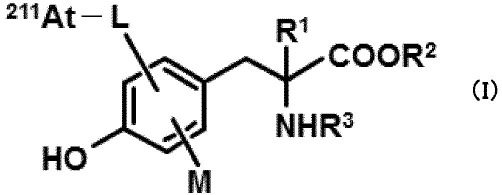
   in which: L represents a direct bond or an optionally substituted C₁-C₅ alkylene group; M represents a hydrogen atom or a halogen atom; R¹ represents an optionally substituted straight chain or branched chain C₁-C₅ alkyl group; R² represents a hydrogen atom, a straight chain or branched chain C₁-C₅ alkyl group, a C₆-C₁₄ aryl group, or a C₇-C₁₆ arylalkyl group; and R³ represents a hydrogen atom or a straight chain or branched chain C₁-C₃ alkyl group,
   wherein the cancer is associated with expression of LAT1, and is selected from a group consisting of pancreatic cancer, leukemia, colorectal cancer, lung cancer, prostate cancer, stomach cancer, breast cancer, kidney cancer, laryngeal cancer, esophageal cancer, liver cancer, lymphoma, myeloma, head and neck cancer, ovarian cancer, bladder cancer, childhood cancer, childhood leukemia, and a brain tumor;
<2> The pharmaceutical composition for use according to <1> described above, in which: L represents a direct bond; M represents a hydrogen atom or a halogen atom; R¹ represents an optionally substituted C₁-C₅ alkyl group; R² represents a hydrogen atom or a C₁-C₅ alkyl group; and R³ represents a hydrogen atom;
<3> The pharmaceutical composition for use according to <1> described above, in which the compound is any of compounds having following structures or a combination of these compounds, 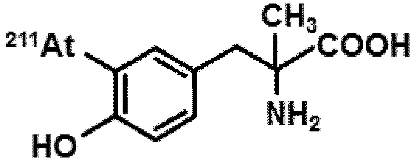 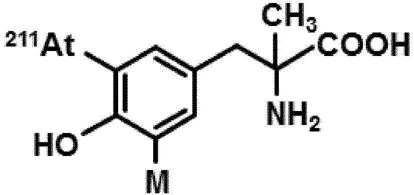 in which M represents I or Br;
<4> The pharmaceutical composition for use according to any of <1> to <3> described above, further including a stabilizer for increasing stability of the compound in a living body, in which the stabilizer is selected from a group consisting of ascorbic acid, an alkali metal salt of ascorbic acid, and an alkaline earth metal salt of ascorbic acid;
<5> The pharmaceutical composition for use according to any of <1> to <4>, further including a probe compound for positron emission tomography (PET); and
<6> A kit including one or more compounds having a structure represented by a following formula (I) or a pharmaceutically acceptable salt thereof; and ascorbic acid or an ascorbic acid salt, 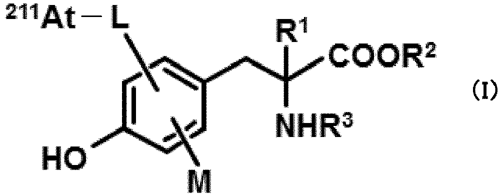 wherein: L represents a direct bond or an optionally substituted C₁-C₅ alkylene group; M represents a hydrogen atom or a halogen atom; R¹ represents an optionally substituted, straight chain or branched chain C₁-C₅ alkyl group; R² represents a hydrogen atom, a straight chain or branched chain C₁-C₅ alkyl group, a C₆-C₁₄ aryl group, or a C₇-C₁₆ arylalkyl group; and R³ represents a hydrogen atom or a C₁-C₃ straight chain or branched chain alkyl group.

In another aspect, regarding a method for producing a ²¹¹At-labeled amino acid derivative,
the present invention provides:
<7> A method for producing a compound represented by a following formula (I), 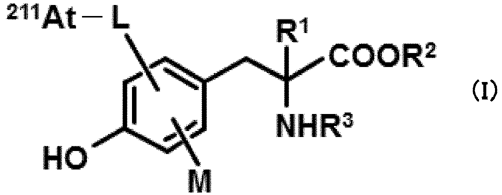
   in which: L represents a direct bond or an optionally substituted C₁-C₅ alkylene group; M represents a hydrogen atom or a halogen atom; R¹ represents an optionally substituted straight chain or branched chain C₁-C₅ alkyl group; R² represents a hydrogen atom, a straight chain or branched chain C₁-C₅ alkyl group, a C₆-C₁₄ aryl group, or a C₇-C₁₆ arylalkyl group; and R³ represents a hydrogen atom or a C₁-C₃ straight chain or branched chain alkyl group,
   the method including the following steps:
      step A) for adding a mercury compound to a compound represented
      by a following formula (Ia), 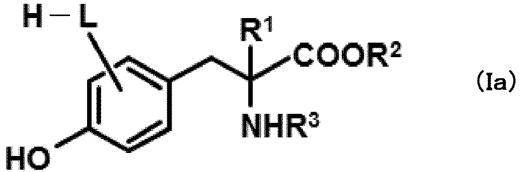
      in which L, R¹, R², and R³ have the same definition as in the above formula (I),
      to obtain a precursor compound represented by a following formula (Ib-1) or a formula (Ib-2), 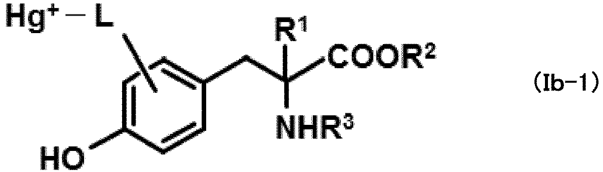 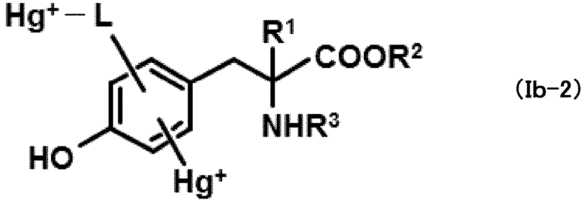
      in which L, R¹, R², and R³ have the same definition as in the above formula (I);
      step B) for adding ²¹¹At and a halide constituted by a halogen element other than astatine to the precursor compound represented by the formula (Ib-1) or the formula (Ib-2) obtained in the step A); and
      step C) for purifying a reaction product obtained in the step B) to obtain the compound represented by the formula (I);
<8> The production method according to <7> described above further including a step for adding ascorbic acid or an ascorbic acid salt after the purification in the step C);
<9> The production method according to <7> or <8>, in which the halide in the step B) is a triiodide;
<10> The production method according to any of <7> to <9>, in which the purification in the step C) is performed by column chromatography using an ion exchange resin; and
<11> The production method according to any of <7> to <10>, in which L represents a single bond.

### Advantageous Effects of Invention

According to the present invention, cancer cells can be treated by radiation of α-rays of the compound specifically and highly accumulated in the cancer cells. While an existing radioactive nuclide used for cancer treatment, such as ¹³¹I, has a longer half-life and emits a beta ray having a long travel distance or the like, ²¹¹At used in the present invention has a short half-life of 7.2 hours and specifically emits an α-ray having a short travel distance. Thus, ²¹¹At can advantageously exhibit more specific cell-killing activity with less adverse effects on normal cells. Further, LAT1 itself is rarely expressed in normal cells, thus it is safe to say that the possibility of having a side effect is negligibly small.

Further, the pharmaceutical composition of the present invention can be administered to a patient of pancreatic cancer, leukemia, or the like by oral administration or injection, and the affected site can be treated by irradiation of α-rays emitted from ²¹¹At selectively accumulated in the affected site. Thus, the pharmaceutical composition is useful in the nuclear medicine therapy for intractable cancer or advanced cancer not suitable for a surgical operation. The cell-killing activity of α-rays is about 100 times higher than that of β-rays, thus the pharmaceutical composition of the present invention using ²¹¹At has an advantage in that regression of the tumor can be expected by single administration over a conventional LAT1 inhibitor which induces nutrient starvation in the cancer cells.

### Brief Description of Drawings

Fig. 1 is an image illustrating stability of a ²¹¹At-labeled amino acid derivative compound (²¹¹At-AAMT) in the presence and absence of ascorbic acid.
Fig. 2 is an image illustrating stability of radioactivity in blood and urine of normal rats (two animals) one hour after intravenous injection of ²¹¹At-AAMT (with ascorbic acid).
Fig. 3 is a graph showing biodistribution of ²¹¹At-AAMT in major organs when ²¹¹At-AAMT is intravenously injected in normal mice with or without ascorbic acid.
Fig. 4 is a graph showing accumulation of ²¹¹At-AAMT in a human pancreatic cancer cell line (PANC-1).
Fig. 5 is a graph comparing accumulation of ²¹¹At-AAMT in cancer cells in the presence and absence of ascorbic acid.
Fig. 6 is a graph showing a cell-killing effect of ²¹¹At-AAMT on the human pancreatic cancer cell line (PANC-1).
Fig. 7 is a graph comparing the cell-killing effect of ²¹¹At-AAMT in the presence and absence of ascorbic acid.
Fig. 8 is graphs showing a change in a tumor size (Fig. 8(A)) and a change in body weight (Fig. 8(B)) of a cancer-bearing mouse to which ²¹¹At-AAMT is administered.
Fig. 9 is SPECT images of ²¹¹At-AAMT (with ascorbic acid, at a dose of 1 MBq each by intravenous injection) in pancreatic cancer (PANC-1) transplanted mice (two animals).
Fig. 10 is SPECT images (20 min to 1 hr) of ²¹¹At-AAMT (without ascorbic acid, at a dose of 1 MBq by intravenous injection) in normal rats.
Fig. 11 is a graph showing the number of nodules in melanoma cell (B16) transplanted mice to which ²¹¹At-AAMT is administered.
Fig. 12 is a graph showing a change in body weight of the melanoma cell (B16) transplanted mice to which ²¹¹At-AAMT is administered.

### Description of Embodiments

Embodiments of the present invention are described below. The embodiments described below do not restrict the scope of the present invention, and changes other than according to the cited examples below may also be implemented as appropriate in a range not compromising the intent of the present invention.

### 1. Definitions

In the present specification, an "alkyl or alkyl group" may be any aliphatic hydrocarbon group including a straight chain, a branched chain, a ring, or any combination thereof. The carbon number of the alkyl group is not particularly limited, and may be, e.g., 1 to 20 (C₁₋₂₀), 1 to 15 (C₁₋₁₅), and 1 to 10 (C₁₋₁₀). In the present specification, alkyl groups may have one or more of any substituent. For example, C₁₋₈ alkyl includes methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, and the like. Alkoxy groups, halogen atoms (the term "halogen atom" means a fluorine atom, a chlorine atom, a bromine atom, or an iodine atom), amino groups, mono- or di-substituted amino groups, substituted silyl groups, or acyl groups and the like can be cited as examples of substituents, but the possible substituents are not thus limited. When an alkyl group has two or more substituents, the substituents may be the same or different. The same applies for the alkyl portions of other substituents which include an alkyl portion (e.g., alkoxy groups, arylalkyl groups, and the like).

In the present specification, an "alkylene" is a divalent group including a straight-chain or branched saturated hydrocarbon, and examples thereof include methylene, 1-methylmethylene, 1,1-dimethylmethylene, ethylene, 1-methylethylene, 1-ethylethylene, 1,1-dimethylethylene, 1,2-dimethylethylene, 1,1-diethylethylene, 1,2-diethylethylene, 1-ethyl-2-methylethylene, trimethylene, 1-methyltrimethylene, 2-methyltrimethylene, 1,1-dimethyltrimethylene, 1,2-dimethyltrimethylene, 2,2-dimethyltrimethylene, 1-ethyltrimethylene, 2-ethyltrimethylene, 1,1-diethyltrimethylene, 1,2-diethyltrimethylene, 2,2-diethyltrimethylene, 2-ethyl-2-methyltrimethylene, tetramethylene, 1-methyltetramethylene, 2-methyltetramethylene, 1,1-dimethyltetramethylene, 1,2-dimethyltetramethylene, 2,2-dimethyltetramethylene, 2,2-din-propyltrimethylene, and the like.

In the present specification, "aryl or aryl group" may refer to any monocyclic or condensed polycyclic aromatic hydrocarbon group, and may include one or more hetero atoms (e.g., oxygen atoms, nitrogen atoms, sulfur atoms, or the like) as annular atoms. In this case, the group is sometimes referred to as a "heteroaryl" group or a "heteroaromatic" group. Bonding may occur at all possible positions whether the aryl has a monocyclic or a condensed ring structure. In the present specification, an aryl group may have one or more of any substituent on the ring thereof. Alkoxy groups, halogen atoms, amino groups, mono- or di-substituted amino groups, substituted silyl groups, or acyl and the like can be cited as examples of substituents, but the possible substituents are not thus limited. When an aryl group has two or more substituents, the substituents may be the same or different. The same applies for the aryl portions of other substituents which include an aryl portion (e.g., aryloxy groups, arylalkyl groups, and the like).

In the present specification, the term "arylalkyl" signifies an alkyl substituted with an abovementioned aryl. The arylalkyl may have one or more of any substituent. Alkoxy groups, halogen atoms, amino groups, mono- or di-substituted amino groups, substituted silyl groups, or acyl groups and the like can be cited as examples of substituents, but the possible substituents are not thus limited. When an acyl group has two or more substituents, the substituents may be the same or different. Representatively, arylalkylbenzyl, p-methoxybenzyl, and the like can be mentioned.

In the present specification, when a certain functional group is defined as being "optionally substituted", a type of substituent, a substitution position, and the number of substituents are not particularly limited. When there are two or more substituents, they may be the same or different. Examples of the substituent can include an alkyl group, an alkoxy group, a hydroxyl group, a carboxyl group, a halogen atom, a sulfo group, an amino group, an alkoxycarbonyl group, and an oxo group, without being limited thereto. Further substituents may be present in these substituents. Examples of such a case can include a halogenated alkyl group without being limited thereto.

The term "amide" used in the present specification includes both RNR'CO-- (Alkylaminocarbonyl- when R is an alkyl) and RCONR'-- (alkylcarbonylamino- when R is an alkyl) .

The term "ester" used in the present specification includes both ROCO-- (alkoxycarbonyl- when R is an alkyl) and RCOO-- (alkylcarbonyloxy- when R is an alkyl).

In the present specification, a certain substituent may form a ring structure with another substituent, and in such cases where substituents are bonded, a person skilled in the art can understand the specific substitution, e.g., the formation of a bond to hydrogen. Consequently, when specific substituents are described as together forming a ring structure, a person skilled in the art can understand that the ring structure can be formed by a normal chemical reaction and easily be generated. Such ring structures as well as the processes for forming the same are within the cognizance of a person skilled in the art. Further, the heterocyclic structure may include any substituent on the ring.

### 2. ²¹¹At-labeled amino acid derivative

A pharmaceutical composition of the present invention is characterized by including a compound having a structure in which ²¹¹At is introduced as a substituent into an amino acid derivative having an affinity with an amino acid transporter LAT1 (in the present specification, also referred to as a "²¹¹At-labeled amino acid derivative compound", or a pharmaceutically acceptable salt thereof.

It has been known that LAT1 is one of amino acid transporters which are membrane proteins required for cellular uptake of neutral branched chain amino acids and aromatic amino acids, and is specifically expressed in cancer cells. LAT1 has a role in supplying amino acids as nutrients in cancer tissues. In the present specification, the term "amino acid derivative having affinity with LAT1" refers to an amino acid derivative that can be taken up by LAT1, more preferably, an amino acid derivative having properties of being taken up more easily by LAT1 than amino acid transporters (e.g., LAT2) in normal cells. As the amino acid derivative having an affinity with LAT1, an aromatic amino acid derivative is preferably mentioned, and as a typical example thereof, a tyrosine derivative such as α-methyltyrosine can be mentioned.

On the other hand, ²¹¹At is a radioactive isotope of astatine (At), which is an element belonging to halogen. ²¹¹At decays to stable lead (²⁰⁷Pb) with the emission of a high-energy α-ray having cell-killing activity. A half-life of ²¹¹At is 7.2 hours. That is, when ²¹¹At, which has a short half-life and high cell-killing activity, is used as a substituent for labeling and brought to an affected site, it becomes possible to efficiently destroy the cancer tissue. Further, when ²¹¹At having such a feature is used as a substituent for labeling a LAT1-affinity compound, it becomes possible to provide a therapeutic effect in which α-ray irradiation can be applied using ²¹¹At that is specifically accumulated in the cancer cells expressing LAT1 with almost no risk of having side effects as LAT1 itself is rarely expressed in normal cells.

In the present invention, the above "compound having a structure in which ²¹¹At is introduced as a substituent into an amino acid derivative having an affinity with an amino acid transporter LAT1" has a structure represented by the following formula (I). The compound represented by the formula (I) has tyrosine as a basic structure. Note that one type of the compound represented by the formula (I) may be solely used, or, in a preferred embodiment, two or more types of the compounds represented by the formula (I) can also be used in combination.

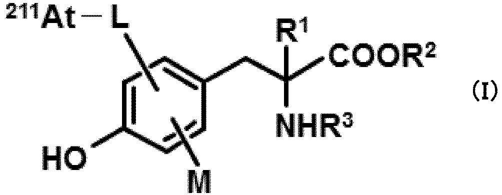

L represents a direct bond or an optionally substituted C₁-C₅ alkylene group; M represents a hydrogen atom or a halogen atom; R¹ represents an optionally substituted straight chain or branched chain C₁-C₅ alkyl group; R² represents a hydrogen atom, a straight chain or branched chain C₁-C₅ alkyl group, a C₆-C₁₄ aryl group, or a C₇-C₁₆ arylalkyl group; and R³ represents a hydrogen atom or a straight chain or branched chain C₁-C₃ alkyl group.

Preferably, L represents a direct bond; R¹ represents an optionally substituted C₁-C₅ alkyl group; R² represents a hydrogen atom or a C₁-C₅ alkyl group; and R³ represents a hydrogen atom.

A halogen atom in M is preferably iodine (I) or bromide (Br), more preferably, I. In a preferred embodiment, M is a hydrogen atom or I.

²¹¹At-L, which can exist at any position on the benzene ring, preferably exists in an ortho position with respect to the OH group. More preferably, ²¹¹At-L can exist in an ortho position with respect to the OH group and in a meta position with respect to the side chain (a linking group to a moiety having R¹, COOR², and NHR³) on the benzene ring. Further, M, which can exist at any position on the benzene ring (any position other than a position where ²¹¹At-L or the OH group exists), preferably can exist in an ortho position with respect to the OH group and in a meta position with respect to ²¹¹At-L (in this case, M is in a meta position with respect to the side chain).

Non-limiting specific examples of the amino acid derivative compound represented by the formula (I) include the following compound (also referred to as "²¹¹At-AAMT" in the present specification). In this compound, ²¹¹At is directly introduced on the benzene ring of α-methyltyrosine.

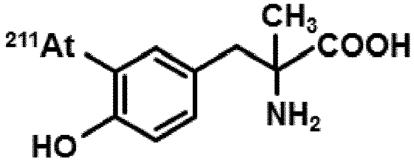

Further, in another preferred embodiment, non-limiting specific examples of the amino acid derivative compound represented by the formula (I) can include the following compound (also referred to as "²¹¹At-AIAMT" in the present specification). In this compound, similarly to the above ²¹¹At-AAMT, ²¹¹At is directly introduced on the benzene ring of α-methyltyrosine. Additionally, it has a structure in which a halogen atom M is introduced in an ortho position with respect to the OH group and in a meta position with respect to ²¹¹At-L. In the formula, M is I or Br.

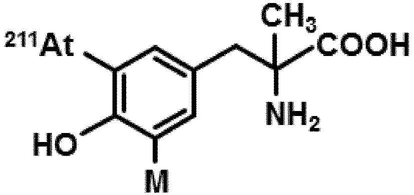

As described above, ²¹¹At-AAMT and ²¹¹At-AIAMT each can be solely used, or these two compounds can be used in combination.

The ²¹¹At-labeled amino acid derivative compound used in the pharmaceutical composition of the present invention can be provided as a form of a pharmaceutically acceptable salt. Such salt is not particularly limited, and base addition salts, acid addition salts, amino acid salts, and the like can be cited as examples thereof. Sodium salts, potassium salts, calcium salts, magnesium salts, and other alkaline earth metal salts; ammonium salts; or triethylamine salts, piperidine salts, morpholine salts, and other organic amine salts can be cited as examples of base addition salts; and hydrochlorides, hydrobromates, sulfates, nitrates, phosphates, and other mineral acid salts; and salts of methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, salicylic acid, and other organic acids can be cited as examples of acid addition salts. Glycinates, asparaginates, glutamates, and the like can be cited as examples of amino acid salts. The salt may also be an aluminum salt or other metal salt.

The ²¹¹At-labeled amino acid derivative compound used in the pharmaceutical composition of the present invention can be in a D form or an L form. However, it is preferably in an L form. Further, the ²¹¹At-labeled amino acid derivative compound may have one or two or more asymmetric carbons depending on the types of substituents, and it may exist as a stereoisomer such as an optical isomer or a diastereomer. A stereoisomer in a pure form, any mixture of stereoisomers, racemate, and the like are all encompassed within the scope of the present invention.

The ²¹¹At-labeled amino acid derivative compound or a pharmaceutically acceptable salt thereof used in the pharmaceutical composition of the present invention may also exist as a hydrate or a solvate. Any of these substances are encompassed within the scope of the present invention. The type of solvent for forming a solvate is not particularly limited, but ethanol, acetone, isopropanol, and other solvents can be cited as examples thereof.

### 3. Pharmaceutical composition

The pharmaceutical composition of the present invention includes the ²¹¹At-labeled amino acid derivative compound or a pharmaceutically acceptable salt thereof. Here, the term "composition" subsumes not only products including active components and inactive components (pharmaceutically acceptable excipients) for constituting a carrier, but also any product directly or indirectly generated as a result of association, complexing, or aggregation of any two or more components, as a result of dissociation of one or more components, or as a result of another type of reaction or interaction of one or more components. The pharmaceutical composition of the present invention can include a combination of two or more kinds of compounds corresponding to the "²¹¹At-labeled amino acid derivative compound".

In the present invention, the pharmaceutical composition is for use in the treatment of cancer in which LAT1 is expressed in cancer cells. In particular, the pharmaceutical composition is for use in the treatment of pancreatic cancer, leukemia (including both myeloid leukemia and lymphocytic leukemia), colorectal cancer, lung cancer, prostate cancer, stomach cancer, breast cancer, kidney cancer, laryngeal cancer, esophageal cancer, liver cancer, lymphoma, myeloma, head and neck cancer, ovarian cancer, bladder cancer, childhood cancer, childhood leukemia, or a brain tumor. The pharmaceutical composition can be preferably used for intractable cancer or advanced cancer that is difficult to be treated by a surgical treatment.

Further, the pharmaceutical composition of the present invention may further include a stabilizer for the purpose of reducing degradation of the compound by stabilizing the bond between ²¹¹At and a carbon atom and enhancing stability of the compound in the living body in addition to the ²¹¹At-labeled amino acid derivative compound as an active ingredient. As such a stabilizer, a compound having reducing activity can be typically mentioned. Examples of the stabilizer can include ascorbic acid, an alkali metal salt of ascorbic acid, an alkaline earth metal salt of ascorbic acid, cysteine, glutathione, gentisic acid, and glucose. Of these, ascorbic acid, an alkali metal salt of ascorbic acid, or an alkaline earth metal salt of ascorbic acid is preferable, and ascorbic acid or sodium ascorbate is more preferable. As shown in the following Examples, in the present invention, it has been surprisingly found that using the ²¹¹At-labeled amino acid derivative compound and the reducing agent such as ascorbic acid in combination can not only stabilize the compound for a long period of time as described above but also increase stability of the compound in the living body even after the compound is taken up in the living body, thereby making it possible to improve accumulation of the compound in the cancer cells. These are unconventional characteristics not found in the related art.

The pharmaceutical composition of the present invention can further include a probe compound capable of detecting a cancer tissue. Such a probe compound is preferably a probe compound for positron emission tomography (PET). As a probe compound for PET (PET agent), a publicly known probe compound for PET in the related art can be used. For example, a PET agent labeled with fluorine (¹⁸F) can be used. Using both the ²¹¹At-labeled amino acid derivative compound capable of performing α-ray treatment and the probe compound capable of detecting the cancer tissue makes it possible to monitor delivery of the agent to the affected site, the condition of the affected site after administration, and the like during the treatment.

The type of pharmaceutical composition is not particularly limited, and can be prepared as a pharmaceutical composition for oral administration in a form such as granules, fine granules, a powder, a hard capsule, a soft capsule, a syrup, an emulsion, a suspension, or a liquid, or as a pharmaceutical composition for parenteral administration in the form of an injection such as for intravenous injection, intramuscular injection, or subcutaneous injection, or in a form such as drops, a percutaneous absorption preparation, a transmucosal absorption preparation, nasal drops, an inhalant, or a suppository. These preparations are prepared in accordance with the usual methods. A preferable dosage form is a liquid preparation for oral administration or injection.

Such a liquid preparation is formulated by dissolving the ²¹¹At-labeled amino acid derivative compound of the present invention in water. However, if necessary, it may be dissolved in a physiological saline solution or a glucose solution, or a buffer or a preservative may be added to it. Further, as described above, the reducing agent such as ascorbic acid can be further included. Particularly, to manufacture an injection, the active ingredient may be dissolved in distilled water for injection together with hydrochloric acid, sodium hydroxide, lactose, lactic acid, sodium, sodium hydrogen phosphate, sodium dihydrogen phosphate, or another pH adjuster, and sodium chloride, glucose, or another isotonizing agent as needed, and sterile-filtered and filled into an ampoule, or mannitol, dextrin, cyclodextrin, gelatin, or the like may be further added and the product vacuum freeze-dried to obtain an injection to be dissolved at the time of use thereof. Lecithin, polysorbate 80, polyoxyethylene hardened castor oil, or the like may also be added to the active ingredient and emulsified in water to obtain an emulsion for injection.

The types of additives for preparation used in manufacturing the pharmaceutical composition, the ratios of additives for preparation with respect to the active ingredient, or the method for producing the pharmaceutical composition can appropriately be selected by a person skilled in the art in accordance with the form of the composition. Inorganic or organic substances, or solid or liquid substances may be used as additives for preparation, and additives for preparation may generally be blended in the range of 1 wt% to 90 wt% with respect to the weight of the active ingredient.

The dosage and number of doses of the pharmaceutical composition of the present invention are not particularly limited, and can appropriately be selected by the judgment of a physician in accordance with the purpose for preventing and/or treating the worsening or progress of the disease to be treated, the type of disease, the body weight or age of the patient, the degree of serious ness of the disease, and other conditions.

In some cases, the pharmaceutical composition can be provided in a form of a kit including the ²¹¹At-labeled amino acid derivative compound of the present invention and the reducing agent such as ascorbic acid or an ascorbic acid salt. In such a case, the pharmaceutical composition is used as an aqueous solution in which the compound and the reducing agent such as ascorbic acid are mixed at the time of use.

### 4. Production method of ²¹¹At-labeled amino acid derivative

In another embodiment, the present invention also relates to a method for producing the ²¹¹At-labeled amino acid derivative. More specifically, the production method of the present invention is a method for producing the compound represented by the above formula (I) and characterized by including the following steps A) to C).

### Step A):

A step for adding a mercury compound to a compound represented by the following formula (Ia) to obtain a precursor compound represented by the following formula (Ib-1) or formula (Ib-2). 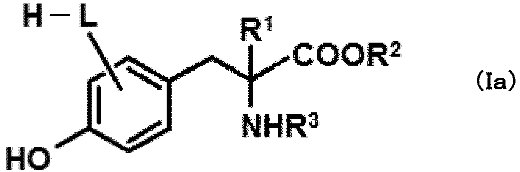 in which L, R¹, R², and R³ have the same definition as in the above formula (I); 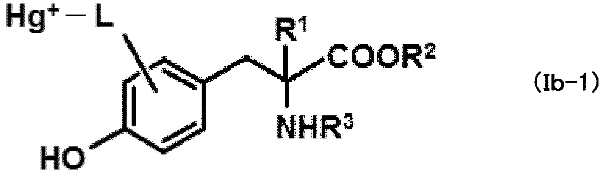 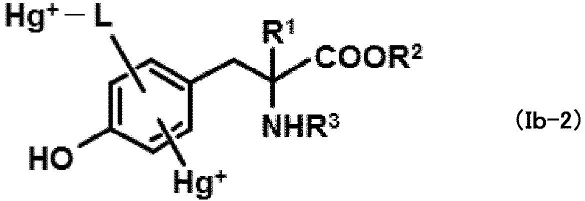 in which L, R¹, R², and R³ have the same definition as in the above formula (I).

The step A) is a step in which a mercury compound is added to an amino acid derivative of the formula (Ia) serving as a starting substance not including ²¹¹At to obtain a precursor in which an aromatic hydrocarbon-mercury bond is formed. In this step, the mercury compound is not particularly limited as long as including a mercury ion (Hg²⁺). For example, HgSO₄ or Hg(OAc)₂ can be used. The step A) is preferably performed under an acidic condition by adding sulfuric acid or the like. Further, the precursor compound represented by the formula (Ib-1) or the formula (Ib-2) can be selectively produced by changing the amount of the mercury compound used in the step A). More specifically, the precursor compound of the formula (Ib-2) in which mercury ions (Hg) are introduced in two positions on the benzene ring can be produced by increasing the mole ratio of the mercury compound with respect to the compound represented by the formula (Ia) serving as a raw substance, typically by performing a reaction under the condition in which the mole ratio [mercury compound/compound represented by formula (Ia)] is 1 or greater. Note that both the precursor compounds of the formula (Ib-1) and the formula (Ib-2) can be produced under some condition. However, such a step is also permitted as long as the compound corresponding to the formula (I) is produced via the following steps B) and C).

### Step B):

The step B) is a step for adding ²¹¹At and a halide constituted by a halogen element other than astatine to the precursor compound represented by the formula (Ib-1) or the formula (Ib-2) obtained in the step A). This reaction is performed to introduce ²¹¹At in replacement of Hg⁺ in the formula (Ib) by using a halide ion as a carrier.

²¹¹At used in the step B) can be obtained from bismuth (Bi) by using an accelerator (cyclotron). More specifically, ²¹¹At is produced by a nuclear reaction of ²⁰⁹Bi(α, 2n)²¹¹At in which bismuth is irradiated with a helium particle accelerated by 28 MeV or more using cyclotron. ²¹¹At is captured and dissolved in water or an organic solvent to obtain a raw liquid of ²¹¹At, which can be used for the reaction in the step B).

The halide used in the step B) is preferably a triiodide. An alkali metal triiodide salt such as KI₃ can be more preferably used. Further, after addition of the triiodide, the corresponding monoiodide can be further added. For example, ²¹¹At and KI₃ are added in the same time and then KI can be further added after ²¹¹At and KI₃ are stirred for a predetermined time.

### Step C):

The step C) is a step for purifying the reaction product obtained in the above step B) to obtain the compound represented by the above formula (I). This step is for removing a byproduct other than the desired compound represented by the formula (I) from the product in the step B).

Purification in the step C) is preferably performed by column chromatography using an ion exchange resin. For example, the desired product can be isolated and purified by performing cation exchange column chromatography and then performing anion exchange column chromatography. Note that, as the ion exchange resin, an ion exchange resin conventionally used in the related art can be used

Further, in a preferred embodiment, a step for adding ascorbic acid or an ascorbic acid salt can be further included after the purification in the step C). This reduces degradation of the purified compound of the formula (I), making it possible to store the compound for a long period of time.

Regarding reaction conditions such as a solvent and a reaction temperature in each step of the production method of the present invention described above, a representative example thereof will be described in detail in the following Examples. However, the reaction conditions are not necessarily limited thereto, and a person skilled in the art could appropriately select the reaction conditions on the basis of knowledge common in the field of organic synthesis.

### Examples

Below, the present invention will be described in further detail using Examples. However, the present invention is not limited to these Examples.

### Example 1

### 1-1. Synthesis of ²¹¹At-labeled amino acid derivative compound (²¹¹At-AAMT)

### (1) Preparation of ²¹¹At aqueous solution

A target substance Bismuth was irradiated with a helium particle (28 MeV, 1 to 2 µA) accelerated by cyclotron to cause a nuclear reaction of ²⁰⁹Bi(α, 2n)²¹¹At, thereby producing ²¹¹At in the target substance. The target substance including ²¹¹At was heated to 850°C in an electric heater to be melted. The transpired ²¹¹At was dissolved in a small amount of water (0.1 to 2 mL) to prepare a ²¹¹At aqueous solution having a radioactivity concentration of about 5 MBq/ml.

### (2) ²¹¹At labeling reaction

α-methyl-L-tyrosine (22 µmol) and HgSO₄ (20 µmol) were added to 0.5 mL of a H₂SO₄ aqueous solution (0.2 M), and the resulting mixture was stirred at room temperature for 2 hours. NaCl (45 µmol) was added to the reaction solution and the reaction solution was stirred for 5 minutes. To the reaction mixture, 100 µL of the ²¹¹At aqueous solution (about 5 MBq/ml) obtained in the above (1) and 5 µL of 1M KI₃ were added and the reaction solution was stirred for 30 minutes. Subsequently, an appropriate amount of KI was added to the reaction mixture until the suspension solution became transparent, thereby terminating the reaction. The reaction solution thus obtained was passed through a cation column (Dowex^{™} 50 W x 8, 100-200 mesh, 0.2 M NH₃ aqueous solution) and then an anion column (Dowex^{™} 1 x 8, 50-100 mesh, 2% AcOH aqueous solution) to remove salts, thereby obtaining the following compound (²¹¹At-AAMT). The yield was from 60 to 80%.

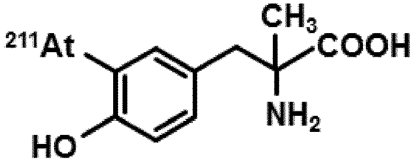

### 1-2. Synthesis of ²¹¹At-labeled amino acid derivative compound (²¹¹At-AIAMT)

### (1) Preparation of ²¹¹At aqueous solution

The ²¹¹At aqueous solution having a radioactivity concentration of about 5 MBq/ml was prepared by the same procedure as described above.

### (2) ²¹¹At labeling reaction

α-methyl-L-tyrosine (22 µmol) and HgSO₄ (100 µmol) were added to 0.5 mL of a H₂SO₄ aqueous solution (0.2 M), and the resulting mixture was stirred at room temperature for 2 hours. NaCl (45 µmol) was added to the reaction solution and the reaction solution was stirred for 5 minutes. To the reaction mixture, 100 µL of the ²¹¹At aqueous solution (about 5 MBq/ml) obtained in the above (1) and 5 µL of 1M KI₃ were added and the reaction solution was stirred for 30 minutes. Subsequently, an appropriate amount of KI was added to the reaction mixture until the suspension solution became transparent, thereby terminating the reaction. The reaction solution thus obtained was passed through a cation column (Dowex^{™} 50 W x 8, 100-200 mesh, 0.2 M NH₃ aqueous solution) and then an anion column (Dowex^{™} 1 x 8, 50-100 mesh, 2% AcOH aqueous solution) to remove salts, thereby obtaining the following compound (²¹¹At-AIAMT). The yield was from 24.5 to 41.5%.

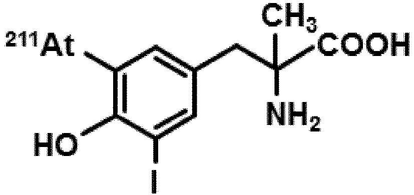

### Example 2

### 2. Stability of ²¹¹At-labeled amino acid derivative compound (²¹¹At-AAMT)

### (1) In vitro stability

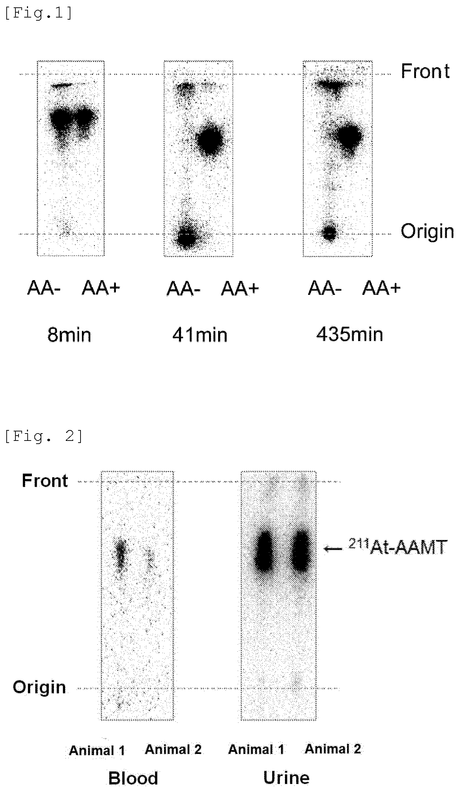

The stability of ²¹¹At-AAMT synthesized in Example 1 was compared in the presence and absence of sodium ascorbate using thin-layer chromatography (TLC). The result is shown in Fig. 1.

As a result, it was found that, in the absence of sodium ascorbate, almost all of ²¹¹At-AAMT was degraded in about 40 minutes, while, in the presence of sodium ascorbate, ²¹¹At-AAMT could stably exist even after the lapse of seven or more hours. This shows that the presence of ascorbic acid, which is a reducing agent, can stabilize ²¹¹At-AAMT by reducing dissociation of aromatic carbon-²¹¹At.

### (2) In vivo (inside of mouse living body) stability

²¹¹At-AAMT to which ascorbic acid was added at a final concentration of 1% was intravenously injected in normal rats, and, after one hour, blood and urine were collected and analyzed by thin-layer chromatography (TLC). As a thin layer plate, silica gel G60F254 (Merck KGaA) was used, and, as a development solvent, a mixture of butanol : water : acetic acid (4 : 1 : 1) was used (Nacalai Tesque, Inc.). The thin layer plate after development was analyzed by using a bioimaging analysis apparatus (Typhoon 7000, GE Healthcare) (Fig. 2). As a result, only ²¹¹At-AAMT was detected in both the blood and the urine, but free ²¹¹At was not observed. That is, it was shown that ²¹¹At-AAMT to which ascorbic acid was added could stably exist in the living body without being metabolized or degraded.

### (3) Effect of addition of ascorbic acid on biodistribution of ²¹¹At-AAMT

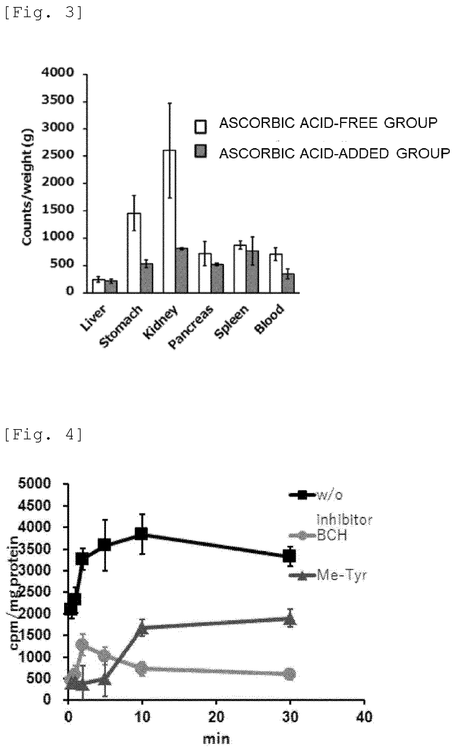

Next, an effect of ascorbic acid on biodistribution of ²¹¹At-AAMT in the mouse body was compared. ddY mice (male, 5 weeks old) purchased from Japan SLC, Inc. were used after being acclimated for one week (6 weeks old at the time of experiment). ²¹¹At-AAMT with 1% ascorbic acid or without ascorbic acid was intravenously injected in normal mice (N = 3), and the mice were dissected after one hour. The organs were all put in polyethylene bags with zipper (A-4, 0.04 mm, SEISANNIPPONSHA Ltd.), and radioactivity of the organs was measured using Wizard² gamma counter (PerkinElmer). The result of ²¹¹At distribution in each major organ is shown in Fig. 3. As a result, it was confirmed that accumulation in the stomach and the kidney was significantly reduced in the 1% ascorbic acid addition group as compared with the ascorbic acid free group. This result shows that adding ascorbic acid has an effect of causing ²¹¹At-AAMT that has been administered to the living body to stably exist in the body.

### Example 3

### 3. Specific accumulation of ²¹¹At-AAMT in cancer cells (in vitro experiment)

A human pancreatic cancer cell line (PANC-1) was treated with ²¹¹At-AAMT and cultured for a predetermined time. Then, the cells were washed to remove unincorporated ²¹¹At-AAMT. The cellular uptake of ²¹¹At-AAMT was measured from the radiation dose remaining in the cells. The result thereof is shown in Fig. 4.

As a result, ²¹¹At-AAMT was taken up immediately after addition of ²¹¹At-AAMT, and the uptake of ²¹¹At-AAMT reached the maximum value in about 10 minutes. Further, the uptake of ²¹¹At-AAMT was reduced by competition with a LAT1 inhibitor, BCH (2-aminobicyclo[2.2.1]heptane-2-carboxylic acid) or methyltyrosine performed in Comparative example. From this result, it was found that ²¹¹At-AAMT was specifically accumulated in the cancer cells via LAT1.

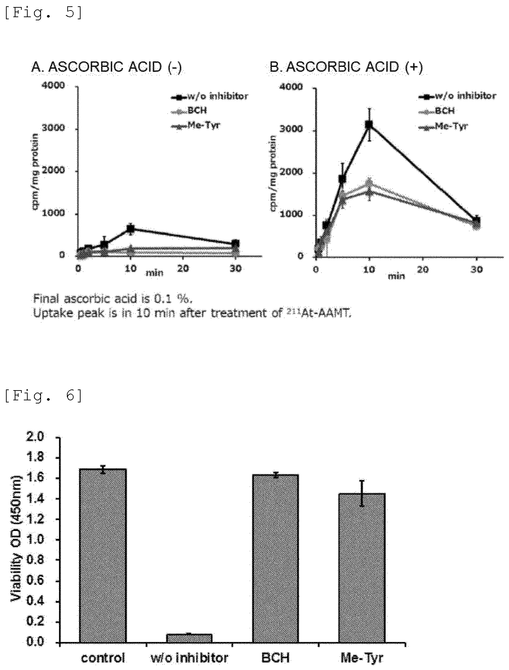

Further, an effect of ascorbic acid on the cellular accumulation of ²¹¹At-AAMT was also examined. The measurement result of the radiation dose in the human pancreatic cancer cell line in the absence and presence of ascorbic acid is shown in Fig. 5. As a result, it was found that accumulation of ²¹¹At-AAMT in the pancreatic cancer cell line (PANC-1) was significantly increased by addition of ascorbic acid (0.1%).

Considering this result and the result of Example 2(3) together, addition of ascorbic acid increased accumulation of ²¹¹At-AAMT in the tumor cells, while addition of ascorbic acid rather reduced accumulation of ²¹¹At-AAMT in normal cells having no tumor, thus it was found that addition of ascorbic acid caused an effect of facilitating clearance of ²¹¹At-AAMT in organs other than the target organ.

### Example 4

### 4. Examination of cell-killing effect of ²¹¹At-AAMT

A cell survival rate of the human pancreatic cancer cell line (PANC-1) administered with ²¹¹At-AAMT under the same conditions as in Example 3 is shown in Fig. 6. As a result, it was confirmed that nearly all of the cancer cells could be killed by ²¹¹At-AAMT. On the other hand, the cellular uptake of ²¹¹At-AAMT was reduced in the presence of the inhibitor BCH or methyltyrosine. As a result, a significant reduction in the cell survival rate was not observed.

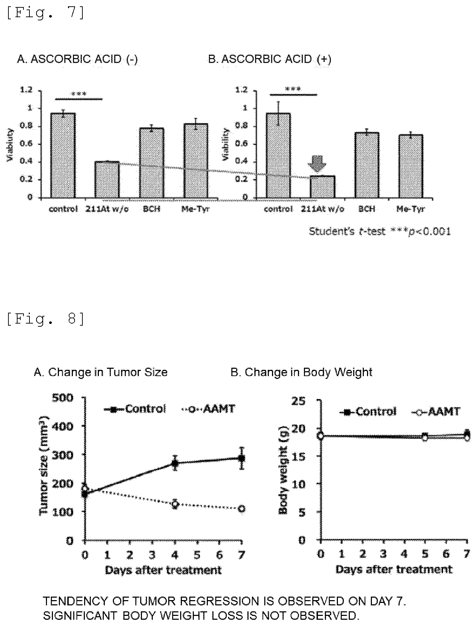

Similarly, an effect of ascorbic acid on the cell-killing effect was compared, and the result thereof is shown in Fig. 7. As a result, it was found that the cancer cell-killing activity of ²¹¹At-AAMT was increased by addition of ascorbic acid. This is because the cellular uptake of ²¹¹At-AAMT in the cancer cells increased about four times by addition of ascorbic acid.

### Example 5

### 5. Administration of ²¹¹At-AAMT to cancer-bearing mouse

A cancer-bearing mouse created by using the human pancreatic cancer cell line (PANC-1) was intravenously administered with ²¹¹At-AAMT at a dose of 0.6 MBq per mouse. A change in the tumor size caused by the administration is shown in Fig. 8(A), while a change in the body weight of the cancer-bearing mouse is shown in Fig. 8(B).

As shown in Fig. 8(A), a regression effect of the tumor was obtained 7 days after the administration. On the other hand, as shown in Fig. 8(B), a substantial reduction of the body weight was not observed 7 days after the administration. This showed that ²¹¹At-AAMT could provide the tumor regression effect without causing significant side effects.

Note that, as a result of performing image analysis of in vivo localization of ²¹¹At-AAMT administered to the normal rat using a gamma camera, accumulation of the radioactivity was observed in the kidney and its surrounding organs, confirming quick excretion of ²¹¹At-AAMT by the kidney.

### Example 6

### 6. Administration of ²¹¹At-AAMT to pancreatic cancer mouse

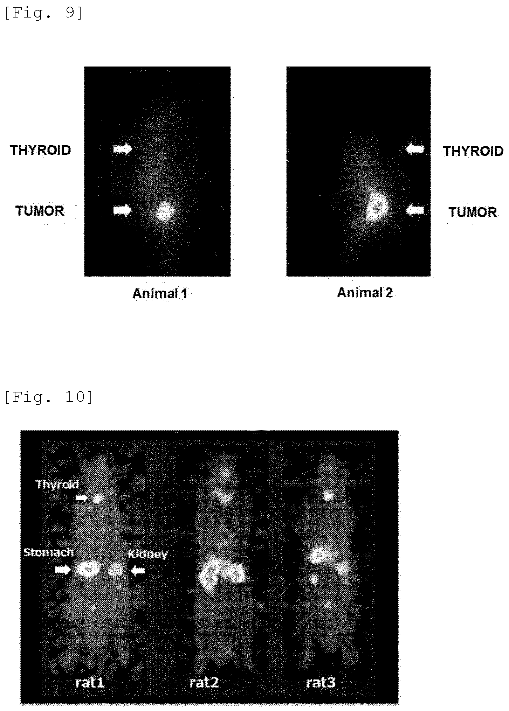

Balb/c-nu/nu mice (female, 5 weeks old) purchased from Japan SLC, Inc. were acclimated in a breeding space for one week. A human pancreas cell line PANC-1 (RIKEN cell bank), which was maintained in an RPMI 1640 medium (Sigma-Aldrich) supplemented with 10% fetal bovine serum (Gibco) and 1% antibiotics (100x, FUJIFILM Wako Pure Chemical Corp.), was adjusted to a concentration of 5 x 10⁶ cells/100µL. Subsequently, the cells were mixed with Matrigel (Growth Factor Reduced, BD) in a 1:1 ratio, and the mixture was transplanted to a subcutaneous region of the hinder back of a mouse using a syringe (FN syringe 27G, Terumo Corp.). Subsequently, after one-month follow-up observation, the mouse having a sufficiently large tumor size was used in the experiment. These pancreatic cancer transplanted mice (animal 1: 25.5 g, animal 2: 30.2 g) were intravenously injected with ²¹¹At-AAMT (with 1% ascorbic acid, at a dose of 1 MBq each), and SPECT imaging was performed after 10 minutes. Imaging was performed using a gamma camera E-Cam (Siemens) (imaging time: 10 minutes, collimator: low energy all purpose, pixel size: 1.2 mm x 1.2 mm, matrix: 256 x 256, zoom: 2-fold, energy window: 79 ± 20%). The obtained result is shown in Fig. 9.

As a result, the tumors were clearly visualized in both cases. On the other hand, the thyroid was not visualized, showing that it was unlikely that free ²¹¹At was produced in the living body under the present conditions. That is, it was found that ²¹¹At-AAMT added with ascorbic acid was highly accumulated in the pancreatic cancer and stable in the living body.

### Example 7

### 7. SPECT imaging in normal rat

Normal rats (Wister, 3 months old, male, 3 animals, body weight of 289.8 ± 5.5 g) were intravenously injected with ²¹¹At-AAMT (without ascorbic acid, 1 MBq), and SPECT imaging was performed after from 20 minutes to 1 hour. Imaging was performed using a gamma camera E-Cam (Siemens) (imaging time: 20 minutes, collimator: low energy all purpose, pixel size: 2.4 mm x 2.4 mm x 2.4 mm (SPECT), matrix: 128 x 128). The obtained result is shown in Fig. 10.

As a result, accumulation of the radioactivity was observed in the thyroid and the stomach in all animals, suggesting that, in the absence of ascorbic acid, ²¹¹At-AAMT was metabolized in the living body to produce free ²¹¹At, which was then accumulated in the thyroid and the stomach.

### Example 8

### 8. Examination of treatment effect of ²¹¹At-AAMT on melanoma metastasis to lung

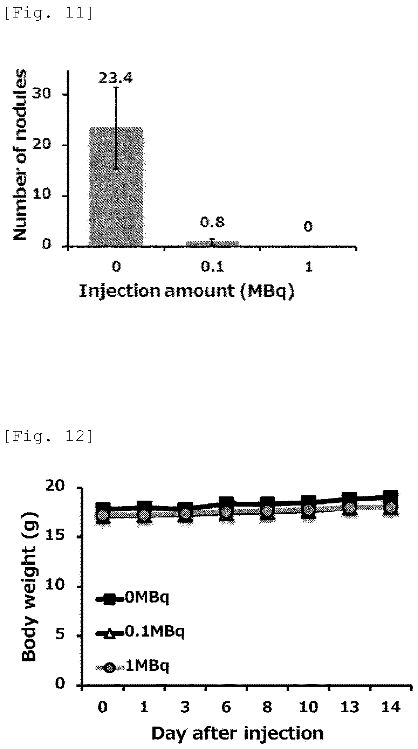

C57BL/6 mice (female, 5 weeks old) purchased from Japan SLC, Inc. were used in the experiment after being acclimated in a breeding space for one week. Melanoma cells B16F10 (a high lung-metastatic cell line cloned from B16, RIKEN cell bank) were maintained in a D-MEM medium (high glucose, Sigma-Aldrich) supplemented with 10% fetal bovine serum (Gibco) and 1% antibiotics (100x, FUJIFILM Wako Pure Chemical Corp.). These cells in an amount of 2 x 10⁵ cells were intravenously transplanted in each mouse. On the following day, the mice (N = 5) was intravenously injected with ²¹¹At-AAMT (with 1% ascorbic acid) at a dose of 0.0 MBq (control group), 0.1 MBq, or 1 MBq. Fourteen days later, the mice were dissected, and the number of nodules spread to the lung was visually counted. The results of the number of the nodules and a change in the body weight thus obtained were shown in Fig. 11 and Fig. 12, respectively.

As a result, it was found that the number of the melanoma nodules in the lung decreased more in the group with more doses of ²¹¹At (Fig. 11). Further, during this period, there was no difference in the body weight change between the ²¹¹At-AAMT group and the control group (Fig. 12). These results show that ²¹¹At-AAMT (with 1% ascorbic acid) is effective in the melanoma metastasis treatment in a dose dependent manner.

## Claims

1. A pharmaceutical composition for use in the treatment of cancer, said composition comprising one or more compounds having a structure represented by a following formula (I) or a pharmaceutically acceptable salt thereof, 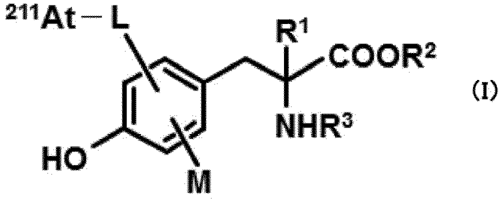
wherein: L represents a direct bond or an optionally substituted C₁-C₅ alkylene group; M represents a hydrogen atom or a halogen atom; R¹ represents an optionally substituted, straight chain or branched chain C₁-C₅ alkyl group; R² represents a hydrogen atom, a straight chain or branched chain C₁-C₅ alkyl group, a C₆-C₁₄ aryl group, or a C₇-C₁₆ arylalkyl group; and R³ represents a hydrogen atom or a straight chain or branched chain C₁-C₃ alkyl group,
wherein the cancer is associated with expression of LAT1, and is selected from a group consisting of pancreatic cancer, leukemia, colorectal cancer, lung cancer, prostate cancer, stomach cancer, breast cancer, kidney cancer, laryngeal cancer, esophageal cancer, liver cancer, lymphoma, myeloma, head and neck cancer, ovarian cancer, bladder cancer, childhood cancer, childhood leukemia, and a brain tumor.

2. The pharmaceutical composition for use according to claim 1, wherein: L represents a direct bond; M represents a hydrogen atom or a halogen atom; R¹ represents an optionally substituted C₁-C₅ alkyl group; R² represents a hydrogen atom or a C₁-C₅ alkyl group; and R³ represents a hydrogen atom.

3. The pharmaceutical composition for use according to claim 1, wherein the compound is any of compounds having following structures or a combination of these compounds, 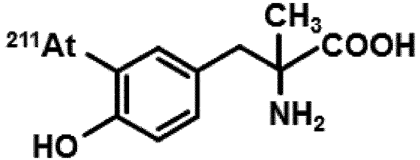 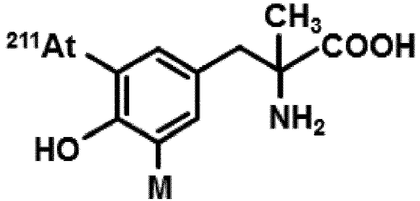 wherein M represents I or Br.

4. The pharmaceutical composition for use according to any of claims 1 to 3, further comprising a stabilizer for increasing stability of the compound in a living body, wherein the stabilizer is selected from a group consisting of ascorbic acid, an alkali metal salt of ascorbic acid, and an alkaline earth metal salt of ascorbic acid.

5. The pharmaceutical composition for use according to any of claims 1 to 4, further comprising a probe compound for positron emission tomography (PET).

6. A kit comprising one or more compounds having a structure represented by a following formula (I) or a pharmaceutically acceptable salt thereof; and ascorbic acid or an ascorbic acid salt, 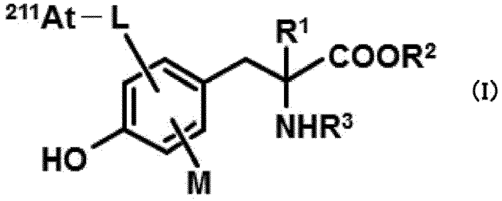 wherein: L represents a direct bond or an optionally substituted C₁-C₅ alkylene group; M represents a hydrogen atom or a halogen atom; R¹ represents an optionally substituted, straight chain or branched chain C₁-C₅ alkyl group; R² represents a hydrogen atom, a straight chain or branched chain C₁-C₅ alkyl group, a C₆-C₁₄ aryl group, or a C₇-C₁₆ arylalkyl group; and R³ represents a hydrogen atom or a C₁-C₃ straight chain or branched chain alkyl group.

7. A method for producing a compound represented by a following formula (I), 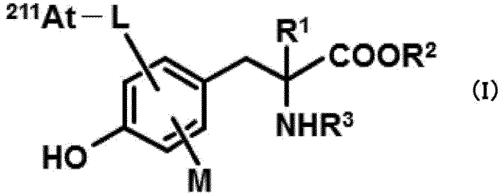
wherein: L represents a direct bond or an optionally substituted C₁-C₅ alkylene group; M represents a hydrogen atom or a halogen atom; R¹ represents a an optionally substituted, straight chain or branched chain C₁-C₅ alkyl group; R² represents a hydrogen atom, a straight chain or branched chain C₁-C₅ alkyl group, a C₆-C₁₄ aryl group, or a C₇-C₁₆ arylalkyl group; and R³ represents a hydrogen atom or a C₁-C₃ straight chain or branched chain alkyl group,
the method comprising the following steps:
step A) for adding a mercury compound to a compound represented by a following formula (Ia), 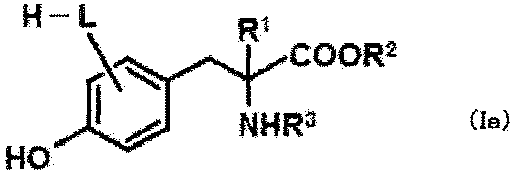
wherein L, R¹, R², and R³ have the same definition as in the above formula (I),
to obtain a precursor compound represented by a following formula (Ib-1) or a formula (Ib-2), 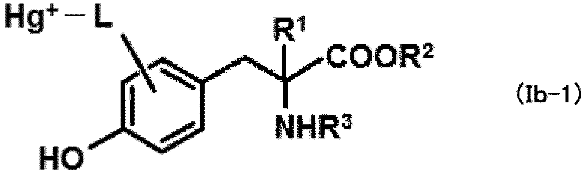 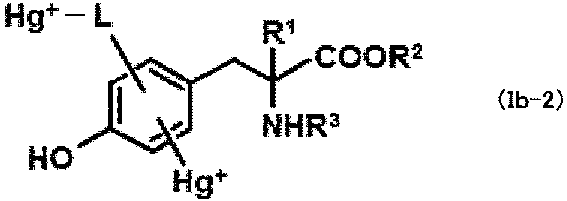
wherein L, R¹, R², and R³ have the same definition as in the above formula (I);
step B) for adding ²¹¹At and a halide constituted by a halogen element other than astatine to the precursor compound represented by the formula (Ib-1) or the formula (Ib-2) obtained in the step A); and
step C) for purifying a reaction product obtained in the step B) to obtain the compound represented by the formula (I).

8. The production method according to claim 7 further comprising a step for adding ascorbic acid or an ascorbic acid salt after the purification in the step C).

9. The production method according to claim 7 or 8, wherein the halide in the step B) is a triiodide.

10. The production method according to any of claims 7 to 9, wherein the purification in the step C) is performed by column chromatography using an ion exchange resin.

11. The production method according to any of claims 7 to 10, wherein L represents a single bond.

## Patentansprüche

1. Pharmazeutische Zusammensetzung zur Verwendung bei der Behandlung von Krebs, wobei die Zusammensetzung eine oder mehrere Verbindungen mit einer durch eine folgende Formel (I) dargestellten Struktur oder ein pharmazeutisch unbedenkliches Salz davon umfasst, 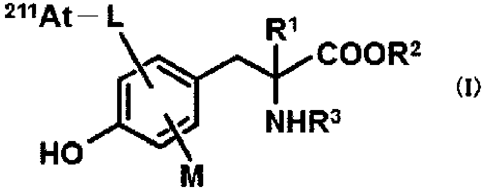
wobei: L eine direkte Bindung oder eine optional substituierte C₁-C₅-Alkylengruppe darstellt; M ein Wasserstoffatom oder ein Halogenatom darstellt; R¹ eine optional substituierte, geradkettige oder verzweigtkettige C₁-C₅-Alkylgruppe darstellt; R² ein Wasserstoffatom, eine geradkettige oder verzweigtkettige C₁-C₅-Alkylgruppe, eine C₆-C₁₄-Arylgruppe oder eine C₇-C₁₆-Arylalkylgruppe darstellt; und R³ ein Wasserstoffatom oder eine geradkettige oder verzweigtkettige C₁-C₃-Alkylgruppe darstellt,
wobei der Krebs mit der Expression von LAT1 assoziiert ist und ausgewählt ist aus einer Gruppe bestehend aus Bauchspeicheldrüsenkrebs, Leukämie, Kolorektalkrebs, Lungenkrebs, Prostatakrebs, Magenkrebs, Brustkrebs, Nierenkrebs, Kehlkopfkrebs, Speiseröhrenkrebs, Leberkrebs, Lymphom, Myelom, Kopf- und Halskrebs, Eierstockkrebs, Blasenkrebs, Krebs im Kindesalter, Leukämie im Kindesalter und einem Gehirntumor.

2. Pharmazeutische Zusammensetzung zur Verwendung gemäß Anspruch 1, wobei: L eine direkte Bindung darstellt; M ein Wasserstoffatom oder ein Halogenatom darstellt; R¹ eine optional substituierte C₁-C₅-Alkylgruppe darstellt; R² ein Wasserstoffatom oder eine C₁-C₅-Alkylgruppe darstellt; und R³ ein Wasserstoffatom darstellt.

3. Pharmazeutische Zusammensetzung zur Verwendung gemäß Anspruch 1, wobei die Verbindung eine Verbindung mit folgenden Strukturen oder eine Kombination dieser Verbindungen ist, 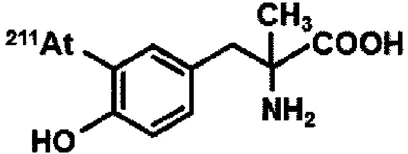 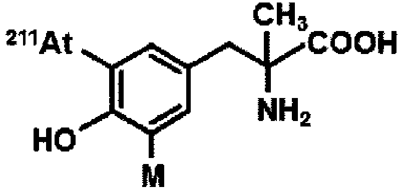 wobei M I oder Br darstellt.

4. Pharmazeutische Zusammensetzung zur Verwendung gemäß einem der Ansprüche 1 bis 3, ferner umfassend einen Stabilisator zur Erhöhung der Stabilität der Verbindung in einem lebenden Körper, wobei der Stabilisator ausgewählt ist aus einer Gruppe bestehend aus Ascorbinsäure, einem Alkalimetallsalz von Ascorbinsäure und einem Erdalkalimetallsalz von Ascorbinsäure.

5. Pharmazeutische Zusammensetzung zur Verwendung gemäß einem der Ansprüche 1 bis 4, ferner umfassend eine Sondenverbindung für die Positronenemissionstomographie (PET).

6. Kit, umfassend eine oder mehrere Verbindungen mit einer durch eine folgende Formel (I) dargestellten Struktur oder ein pharmazeutisch unbedenkliches Salz davon; und Ascorbinsäure oder ein Ascorbinsäuresalz, 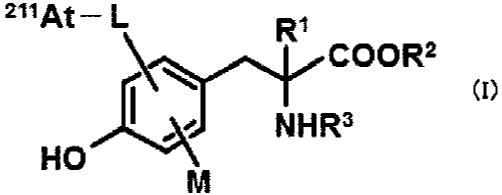 wobei: L eine direkte Bindung oder eine optional substituierte C₁-C₅-Alkylengruppe darstellt; M ein Wasserstoffatom oder ein Halogenatom darstellt; R¹ eine optional substituierte, geradkettige oder verzweigtkettige C₁-C₅-Alkylgruppe darstellt; R² ein Wasserstoffatom, eine geradkettige oder verzweigtkettige C₁-C₅-Alkylgruppe, eine C₆-C₁₄-Arylgruppe oder eine C₇-C₁₆-Arylalkylgruppe darstellt; und R³ ein Wasserstoffatom oder eine geradkettige oder verzweigtkettige C₁-C₃-Alkylgruppe darstellt.

7. Verfahren zur Herstellung einer Verbindung, dargestellt durch eine folgende Formel (I), 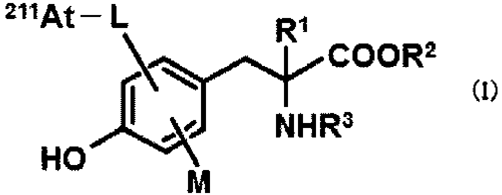
wobei: L eine direkte Bindung oder eine optional substituierte C₁-C₅-Alkylengruppe darstellt; M ein Wasserstoffatom oder ein Halogenatom darstellt; R¹ eine optional substituierte, geradkettige oder verzweigtkettige C₁-C₅-Alkylgruppe darstellt; R² ein Wasserstoffatom, eine geradkettige oder verzweigtkettige C₁-C₅-Alkylgruppe, eine C₆-C₁₄-Arylgruppe oder eine C₇-C₁₆-Arylalkylgruppe darstellt; und R³ ein Wasserstoffatom oder eine geradkettige oder verzweigtkettige C₁-C₃-Alkylgruppe darstellt,
wobei das Verfahren folgende Schritte umfasst:
Schritt A) zum Zugeben einer Quecksilberverbindung zu einer Verbindung, dargestellt durch eine folgende Formel (Ia), 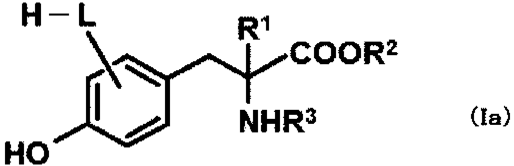
wobei L, R¹, R² und R³ dieselbe Definition wie in der obigen Formel (I) aufweisen,
um eine Vorläuferverbindung zu erhalten, dargestellt durch eine folgende Formel (Ib-1) oder eine Formel (Ib-2), 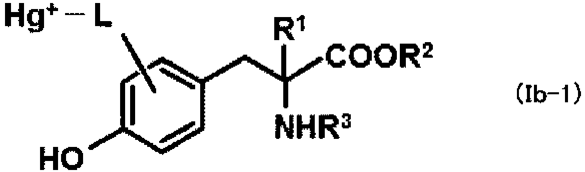 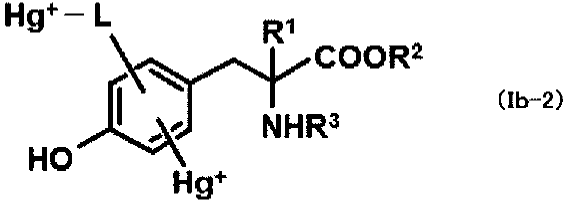
wobei L, R¹, R² und R³ dieselbe Definition wie in der obigen Formel (I) aufweisen;
Schritt B) zum Zugeben von ²¹¹At und einem Halogenid, das aus einem anderen Halogenelement als Astat besteht, zu der Vorläuferverbindung, dargestellt durch die Formel (Ib-1) oder die Formel (Ib-2), die in Schritt A) erhalten wird; und
Schritt C) zum Aufreinigen eines in Schritt B) erhaltenen Reaktionsprodukts, um die durch die Formel (I) dargestellte Verbindung zu erhalten.

8. Herstellungsverfahren gemäß Anspruch 7, ferner umfassend einen Schritt zum Zugeben von Ascorbinsäure oder eines Ascorbinsäuresalzes nach der Aufreinigung in Schritt C).

9. Herstellungsverfahren gemäß Anspruch 7 oder 8, wobei das Halogenid in Schritt B) ein Triiodid ist.

10. Herstellungsverfahren gemäß einem der Ansprüche 7 bis 9, wobei die Aufreinigung in Schritt C) durch Säulenchromatographie unter Verwendung eines Ionenaustauscherharzes durchgeführt wird.

11. Herstellungsverfahren gemäß einem der Ansprüche 7 bis 10, wobei L eine Einfachbindung darstellt.

## Revendications

1. Composition pharmaceutique destinée à être utilisée dans le traitement d'un cancer, ladite composition comprenant un ou plusieurs composés présentant une structure représentée par la formule (I) suivante ou un sel acceptable pharmaceutiquement de ceux-ci, 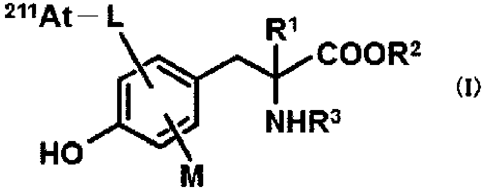
dans laquelle : L représente une liaison directe ou un groupe alkylène en C₁-C₅ éventuellement substitué ; M représente un atome d'hydrogène ou un atome d'halogène ; R¹ représente un groupe alkyle en C₁-C₅ à chaîne droite ou ramifiée éventuellement substitué ; R² représente un atome d'hydrogène, un groupe alkyle en C₁-C₅ à chaîne droite ou ramifiée, un groupe aryle en C₆-C₁₄ ou un groupe arylalkyle en C₇-C₁₆ ; et R³ représente un atome d'hydrogène ou un groupe alkyle en C₁-C₃ à chaîne droite ou ramifiée,
ledit cancer étant associé à l'expression de LAT1 et étant choisi dans un groupe constitué par le cancer du pancréas, la leucémie, le cancer colorectal, le cancer du poumon, le cancer de la prostate, le cancer de l'estomac, le cancer du sein, le cancer du rein, le cancer du larynx, le cancer de l'œsophage, le cancer du foie, le lymphome, le myélome, le cancer de la tête et du cou, le cancer de l'ovaire, le cancer de la vessie, le cancer infantile, la leucémie infantile et une tumeur cérébrale.

2. Composition pharmaceutique destinée à être utilisée selon la revendication 1, dans laquelle : L représente une liaison directe ; M représente un atome d'hydrogène ou un atome d'halogène ; R¹ représente un groupe alkyle en C₁-C₅ éventuellement substitué ; R² représente un atome d'hydrogène ou un groupe alkyle en C₁-C₅ ; et R³ représente un atome d'hydrogène.

3. Composition pharmaceutique destinée à être utilisée selon la revendication 1, dans laquelle le composé est l'un quelconque des composés présentant les structures suivantes ou une combinaison de ces composés, 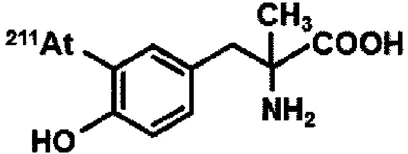 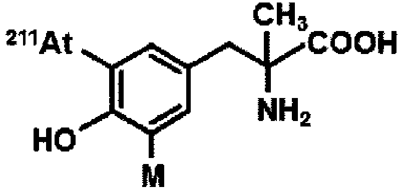 M représentant I ou Br.

4. Composition pharmaceutique destinée à être utilisée selon l'une quelconque des revendications 1 à 3, comprenant en outre un stabilisant destiné à augmenter la stabilité du composé dans un corps vivant, dans laquelle le stabilisant est choisi dans un groupe constitué de l'acide ascorbique, d'un sel de métal alcalin de l'acide ascorbique et d'un sel de métal alcalino-terreux de l'acide ascorbique.

5. Composition pharmaceutique destinée à être utilisée selon l'une quelconque des revendications 1 à 4, comprenant en outre un composé de sonde pour la tomographie par émission de positons (TEP).

6. Kit comprenant un ou plusieurs composés présentant une structure représentée par la formule (I) suivante ou un sel acceptable pharmaceutiquement de ceux-ci ; et l'acide ascorbique ou un sel d'acide ascorbique, 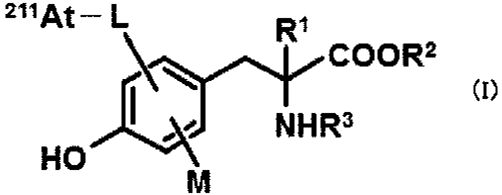 dans lequel : L représente une liaison directe ou un groupe alkylène en C₁-C₅ éventuellement substitué ; M représente un atome d'hydrogène ou un atome d'halogène ; R¹ représente un groupe alkyle en C₁-C₅ à chaîne droite ou ramifiée éventuellement substitué ; R² représente un atome d'hydrogène, un groupe alkyle en C₁-C₅ à chaîne droite ou ramifiée, un groupe aryle en C₆-C₁₄ ou un groupe arylalkyle en C₇-C₁₆ ; et R³ représente un atome d'hydrogène ou un groupe alkyle en C₁-C₃ à chaîne droite ou ramifiée.

7. Procédé pour produire un composé représenté par la formule (I) suivante, 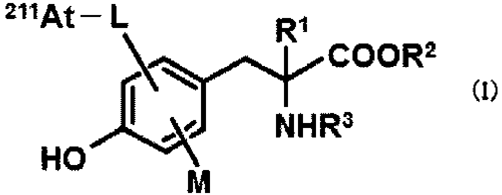
dans lequel : L représente une liaison directe ou un groupe alkylène en C₁-C₅ éventuellement substitué ; M représente un atome d'hydrogène ou un atome d'halogène ; R¹ représente un groupe alkyle en C₁-C₅ à chaîne droite ou ramifiée éventuellement substitué ; R² représente un atome d'hydrogène, un groupe alkyle en C₁-C₅ à chaîne droite ou ramifiée, un groupe aryle en C₆-C₁₄ ou un groupe arylalkyle en C₇-C₁₆ ; et R³ représente un atome d'hydrogène ou un groupe alkyle en C₁-C₃ à chaîne droite ou ramifiée,
le procédé comprenant les étapes suivantes :
étape A) pour l'ajout d'un composé de mercure à un composé représenté par la formule (Ia) suivante, 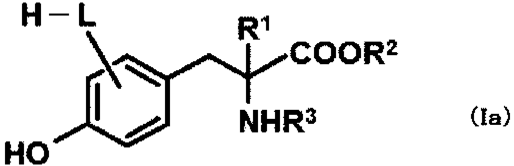
L, R¹, R² et R³ ayant la même définition que dans la formule (I) ci-dessus,
afin d'obtenir un composé précurseur représenté par la formule (Ib-1) ou la formule (Ib-2) suivante, 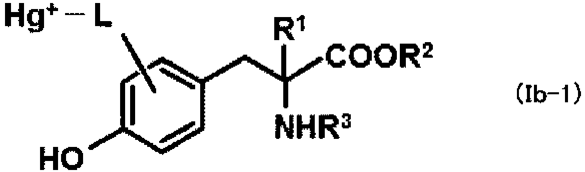 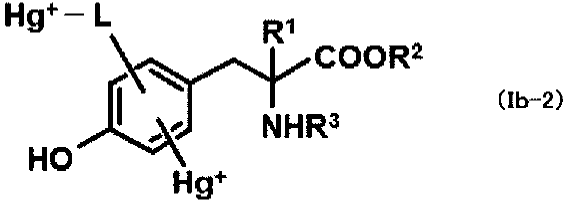
L, R¹, R² et R³ ayant la même définition que dans la formule (I) ci-dessus ;
étape B) pour l'ajout de ²¹¹At et d'un halogénure constitué d'un élément halogène autre que l'astate au composé précurseur représenté par la formule (Ib-1) ou la formule (Ib-2) obtenue à l'étape A) ; et
étape C) pour la purification d'un produit de réaction obtenu à l'étape B) afin d'obtenir le composé représenté par la formule (I).

8. Procédé de production selon la revendication 7, comprenant en outre une étape d'ajout d'acide ascorbique ou d'un sel d'acide ascorbique après la purification dans l'étape C).

9. Procédé de production selon l'une des revendications 7 ou 8, dans lequel l'halogénure dans l'étape B) est un triiodure.

10. Procédé de production selon l'une quelconque des revendications 7 à 9, dans lequel la purification dans l'étape C) est effectuée par chromatographie sur colonne en utilisant une résine échangeuse d'ions.

11. Procédé de production selon l'une quelconque des revendications 7 à 10, dans lequel L représente une liaison simple.
